# EUROPÄISCHE PATENTSCHRIFT

(11) **EP 1 212 392 B1**
(45) Veröffentlichungstag und Bekanntmachung des Hinweises auf die Patenterteilung: **26.10.2011**
(21) Anmeldenummer: 00960540.3
(22) Anmeldetag: 29.08.2000
(51) Int. Cl.: C10M 173/00

(54) **SCHMIERMITTELN AUF POLYSILOXAN-BASIS UND IHRE VERWENDUNG**
USE OF LUBRICANTS WITH A POLYSILOXANE BASE
UTILISATION DE LUBRIFIANTS A BASE DE POLYSILOXANE

(30) Priorität: 07.09.1999 DE 19942536
(43) Veröffentlichungstag der Anmeldung: 12.06.2002
(73) Patentinhaber: ECOLAB INC., St. Paul, MN 55102-2233 (US)
(72) Erfinder: KÜPPER, Stefan, 40764 Langenfeld (DE); SCHNEIDER, Michael, D-41363 Jüchen (DE); GROSSE BÖWING, Walter, 41541 Dormagen (DE); LAUFENBERG, Alfred, 41541 Dormagen (DE); KLUSCHANZOFF, Harald, 40822 Mettmann (DE)
(74) Vertreter: Polypatent
(86) Internationale Anmeldenummer: PCT/EP2000/008395
(87) Internationale Veröffentlichungsnummer: WO 2001/018160

(56) Entgegenhaltungen:
- EP-A- 0 844 299
- WO-A-01/07544
- WO-A-96/08601
- US-A- 4 248 724
- US-A- 4 652 386

## Beschreibung

Die vorliegende Erfindung betrifft die Verwendung von Polysiloxanhaltigen schmiermittelfomnulierungen. Zur Schmietung von Transport dul agen.

In der Lebensmittelindustrie, insbesondere in Gehänkebetrieben werden die in den Abfüllanlagen zu befüllenden Behälter über Transporteure verschiedenster Ausgestaltung und Materialien, beispielsweise über Plattentransportbänder oder kettenartige Anordnungen, die im folgenden allgemein als Transportketten bezeichnet werden sollen, transportiert. Die Transporteure stellen die Verbindung her zwischen den verschiedenen optionalen Behandlungsstufen des Abfüllprozesses wie z. B. Auspacker, Flaschenreinigungsmaschine, Füller, Verschließer, Etikettierer, Einpacker u.a.. Bei den Behältern kann es sich um verschiedenste Formen handeln, insbesondere Glas- und Kunststoffflaschen, Dosen, Gläser, Fässer, Getränkecontainer (KEG), Papier- und Pappbehälter. Um den störungsfreien Betrieb zu gewährleisten, müssen die Transportketten in geeigneter Weise geschmiert werden, so daß eine zu starke Reibung zu den Behältern vermieden wird. Üblicherweise werden zur Schmierung verdünnte wäßrige Lösungen verwendet, die geeignete reibungsvermindernde Wirkstoffe enthalten. Mit den wäßrigen Lösungen werden die Transportketten beispielsweise durch Eintauchen oder durch Besprühen in Kontakt gebracht, wobei man dann von Tauchschmieranlagen oder automatischen Bandschmiersystemen oder zentralen Kettenschmiersystemen spricht

Die bisher als Schmiermittel eingesetzten Kettengleitmittel basieren meist auf Fettsäuren in Form ihrer wasserlöslichen Alkali- oder Alkanolaminsalze oder auf Fettaminen vorzugsweise in Form ihrer organischen oder anorganischen Salze. Während beide Substanzklassen in der Tauchschmierung problemlos anwendbar sind, zeigen sie in den heute üblichen zentralen Kettenschmiersystemen eine Reihe von Nachteilen. So beschreibt die DE-A- 23 13 330 Schmiermittel auf Seifenbasis, die wäßrige Mischungen von C₁₆-C₁₈-Fettsäuresalzen und oberflächenaktiven Substanzen enthalten. Derartige Schmiermittel auf Seifenbasis weisen folgende Nachteile auf:
1. Es kommt zu einer Reaktion mit der Wasserhärte, also den Erdalkali-lonen, und anderen Wasserinhalstoffen unter Bildung schwerlöslicher Metallseifen, den sogenannten primären Erdalkaliseifen.
2. Es kommt zu einer Reaktion zwischen diesen Schmiermitteln auf Seifenbasis und in Wasser oder dem abzufüllenden Gut gelöstem Kohlendioxid.
3. Die so erzeugte Anwendungslösung ist stets keimfördemd.
4. Bei Anwendung von hartem Wasser sind lonenaustauscher zur Wasserenthärtung erforderlich, was eine zusätzliche Keimquelle bedeutet (und deshalb in der Praxis kaum anzutreffen ist), oder aber der Einsatz hoch komplexierungsmittelhaltiger Produkte, was wiederum ökologisch bedenklich ist, ist nötig.
5. Es kommt zu vermehrter Schaumbildung, was insbesondere Probleme am Bottle-Inspector (automatische Flaschenkontrolle) hervorrufen kann und eine stärkere Benetzung der Transportbehältnisse zur Folge hat.
6. Die meisten dieser Produkte sind lösungsmittelhaltig.
7. Die Reinigungswirkung dieser Produkte ist schlecht, so daß eine separate Reinigung notwendig ist.
8. Derartige Schmiermittelzubereitungen auf Seifenbasis zeigen ein pHabhängiges Leistungsverhalten.
9. Schmiermittelzubereitungen auf Seifenbasis zeigen weiterhin eine Wassertemperatur-Abhängigkeit.
10. Schmiermittel auf Seifenbasis zeigen nur eine geringe Lagerstabilität, insbesondere bei niederen Temperaturen.
11. Das in vielen Produkten enthaltene EDTA (Ethylendiamintetraacetat) ist bekanntermaßen nur schlecht biologisch abbaubar.
12. Derartige Schmiermitteizubereitungen auf Seifenbasis sind nicht für alle Transportgüter aus Kunststoff geeignet, da es bei Anwendung dieser Mittel in vielen Fällen zu Spannungsrißkorrosionen am Transportgut kommt.

Neben Schmiermitteln auf Seifenbasis werden hauptsächlich solche auf Basis von Fettaminen verwendet. So beschreibt die DE-A-36 31 953 ein Verfahren zum Schmieren von kettenförmigen Flaschentransportbändem in Getränkeabfüllbetrieben, insbesondere in Brauereien, sowie zum Reinigen der Bänder mittels eines flüssigen Reinigungsmittels, das dadurch gekennzeichnet ist, daß man die kettenförmigen Flaschentransportbänder mit Bandschmiermitteln auf Basis neutralisierter primärer Fettamine, die vorzugsweise 12 bis 18 C-Atome aufweisen und einen ungesättigten Anteil von mehr als 10 % enthalten, schmiert.

Aus der EP-A-0 372 628 sind Fettaminderivate der Formeln 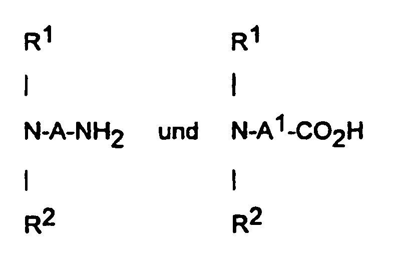 als Schmiermittel bekannt, worin
- R¹: eine gesättigte oder ungesättigte, verzweigte oder lineare Alkylgruppe mit 8 bis 22 C-Atomen;
- R²: Wasserstoff, eine Alkyl- oder Hydroxyalkylgruppe mit 1 bis 4 C-Atomen oder -A-N^{H}2^{;}
- A: eine lineare oder verzweigte Alkylengruppe mit 1 bis 8 C-Atomen; und

A¹ eine lineare oder verzweigte Alkylengruppe mit 2 bis 4 C-Atomen bedeutet.

Darüber hinaus sind aus der DE-A-39 05 548 Schmiermittel auf Basis von N-alkylierten Fettaminderivaten bekannt, die mindestens ein sekundäres und/oder tertiäres Amin enthalten.

Aus der DE-A-42 06 506 sind bekannt
Seifenfreie Schmiermittel auf der Basis von amphoteren Verbindungen, primären, sekundären und/oder tertiären Aminen und/oder Salzen derartiger Amine der allgemeinen Formel (I), (IIa), (IIb), (IIIa), (IIIb), (IIIc), (IVa) und (IVb)

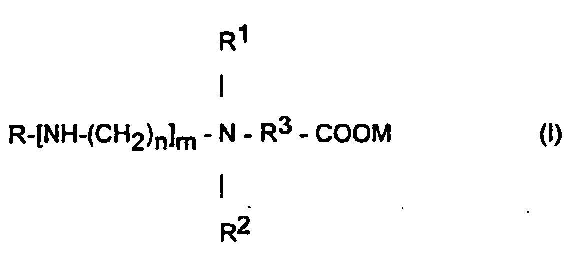

R⁴-NH-R⁵ (IIa)

R⁴-N⁺H₂-R⁵ X⁻ (IIb)

R⁴-NH-(CH₂)₃NH₂ (IIIa)

R⁴-NH-(CH₂)₃N⁺H₃ X⁻ (IIIb)

R⁴-N⁺H₂⁻(CH₂)₃₋N⁺H₃ 2X⁻ (IIIc)

R⁴-NR⁷R⁸ (IVa)

und/oder

R⁴-N⁺HR⁷R⁸ X⁻ (IVb)

wobei
- R: für einen gesättigten oder einfach oder mehrfach ungesättigten, linearen oder verzweigten Alkylrest mit 6 bis 22 C-Atomen, der gegebenenfalls durch -OH,-NH₂, -NH-, -CO-, -(CH₂CH₂O)ₗ- oder -(CH₂CH₂CH₂O)ₗ- substituiert sein kann,
- R¹: für Wasserstoff, einen Alkylrest mit 1 bis 4 C-Atomen, einen Hydroxyalkylrest mit 1 bis 4 C-Atomen oder einen Rest -R³COOM
- R²: nur für den Fall, daß M eine negative Ladung darstellt für Wasserstoff, einen Alkylrest mit 1 bis 4 Atomen, oder einen Hydroxyalkylrest mit 1 bis 4 C-Atomen,
- R³: für einen gesättigten oder einfach oder mehrfach ungesättigten, linearen oder verzweigten Alkylrest mit 1 bis 12 C-Atomen, der gegebenenfalls durch -OH,-NH₂, -NH-, -CO-, -(CH₂CH₂O)ₗ- oder -(CH₂CH₂CH₂O)ₗ- substituiert sein kann,
- R⁴: für einen substituierten oder unsubstituierten, linearen oder verzweigten, gesättigten oder einfach oder mehrfach ungesättigten Alkylrest mit 6 bis 22 C-Atomen, der als Substituenten mindestens einen Amin-, Imin-, Hydroxy-, Halogen- und/oder Carboxyrest aufweisen kann, einen substituierten oder unsubstituierten Phenylrest, der als Substituenten mindestens einen Amin-, Imin-, Hydroxy-, Halogen-, Carboxy- und/oder einen linearen oder verzweigten, gesättigten oder einfach oder mehrfach ungesättigten Alkylrest mit 6 bis 22 C-Atomen aufweisen kann,
- R⁵: für Wasserstoff oder - unabhängig von R⁴ - für einen Rest R⁴,
- X⁻: für ein Anion aus der Gruppe Amidosulfonat, Nitrat, Halogenid, Sulfat, Hydrogencarbonat, Carbonat, Phosphat oder R⁶-COO⁻ steht, wobei

- R⁶: für Wasserstoff, einen substituierten oder unsubstituierten, linearen oder verzweigten Alkylrest mit 1 bis 20 C-Atomen oder Alkenylrest mit 2 bis 20 C-Atomen, die als Substituenten mindestens einen Hydroxy-, Amin- oder Iminrest aufweisen können, oder einen substituierten oder unsubstituierten Phenylrest, der als Substituenten einen Alkylrest mit 1 bis 20 C-Atomen aufweisen kann, steht, und
- R⁷: und R⁸ jeweils unabhängig voneinander für einen substituierten oder unsubstituierten, linearen oder verzweigten Alkylrest mit 1 bis 20 C- Atomen oder Alkenylrest mit 2 bis 20 C-Atomen, die als Substituenten mindestens einen Hydroxy-. Amin- oder Iminrest aufweisen können, oder einen substituierten oder unsubstituierten Phenylrest, der als Substituenten einen Alkylrest mit 1 bis 20 C-Atomen aufweisen kann,
- M: für Wasserstoff, Alkalimetall, Ammonium, einen Alkylrest mit 1 bis 4 C-Atomen, einen Benzylrest oder eine negative Ladung,
- n: für eine ganze Zahl im Bereich von 1 bis 12,
- m: für eine ganze Zahl im Bereich von 0 bis 5 und
- l: für eine Zahl im Bereich von 0 bis 5 steht,
enthaltend Alkyldimethylaminoxide und/oder Alkyloligoglycoside als nichtionische Tenside.

Die EP-B-629 234 offenbart eine Schmiermittelkombination, bestehend aus
a) einer oder mehrerer Verbindungen der Formel 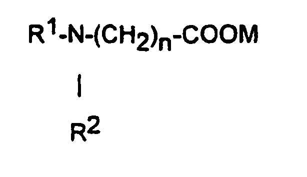 wobei
   - R¹: für einen gesättigten oder einfach oder mehrfach ungesättigten, linearen oder verzweigten Alkylrest mit 6 bis 22 C-Atomen, der gegebenenfalls durch -OH, -NH₂, -NH-, -CO-, Halogen oder einen Carboxylrest substituiert sein kann,
   - R²: für einen Carboxylrest mit 2 bis 7 C-Atomen,
   - M: für Wasserstoff, Alkalimetall, Ammonium, einen Alkylrest mit 1 bis 4 C-Atomen oder einen Benzylrest und
   - n: für eine ganze Zahl im Bereich von 1 bis 6 steht,
b) wenigstens eine organische Carbonsäure ausgewählt aus einbasigen oder mehrbasigen, gesättigten oder einfach oder mehrfach ungesättigten Carbonsäuren mit 2 bis 22 C-Atomen,
c) gegebenenfalls Wasser und Zusatz- und/oder Hilfsstoffe.

Die WO 94/03562 beschreibt ein Schmiermittelkonzentrat auf Basis von Fettaminen und gegebenenfalls üblichen Verdünnungsmitteln oder Hilfs- bzw. Zusatzstoffen, **dadurch gekennzeichnet, daß** es mindestens ein Polyaminderivat eines Fettamins und/oder ein Salz eines derartigen Amins enthält, wobei der Anteil der genannten Polyaminderivate von Fettaminen an der Gesamtformulierung 1 bis 100 Gew.-% beträgt.

Gemäß einer bevorzugten Ausführungsform der WO 94/03562 enthält dieses Schmiermittelkonzentrat mindestens ein Polyaminderivat eines Fettamins der allgemeinen Formel

R-A-(CH₂)ₖ-NH-[(CH₂)ₗ-NH]_{y}-(CH₂)ₘ-NH₂ · (H⁺X⁻)ₙ

wobei
- R: ein substituierter oder unsubstituierter, linearer oder verzweigter, gesättigter oder einfach oder mehrfach ungesättigter Alkylrest mit 6 bis 22 Atomen,

- A: wobei die Substituenten ausgewählt sind aus Amino, Imino, Hydroxy, Halogen und Carboxy, oder ein substituierter oder unsubstituierter Phenylrest, wobei die Substituenten ausgewählt sind aus Amino, lmino, Hydroxy, Halogen, Carboxy und einem linearen oder verzweigten, gesättigten oder einfach oder mehrfach ungesättigten Alkylrest mit 6 bis 22 C-Atomen, ist; entweder für -NH- oder für -O- steht,
- X⁻: ein Anion einer anorganischen oder organischen Säure bedeutet,
- k, I, m: unabhängig voneinander eine ganze Zahl im Bereich von 1 bis 6 ist;
- y: im Falle A = -NH- 0, 1, 2 oder 3 und im Falle A = -O**-** 1, 2, 3 oder 4 ist,
- n: eine ganze Zahl von 0 bis 6 ist.

Die vorliegende Erfindung stellt sich die Aufgabe, bei Verwendung von Silikonhaltigen Formulierungen die Schmierung von Transportbandanlagen zu ermöglichen, den Wasserverbrauch zu senken und eine gute Materialverträglichkeit mit Kunststoff-Gebinden zu erreichen.

Die vorliegende Erfindung betrifft die Verwendung von Formulierungen, die, bezogen auf die gesamte Formulierung, wenigstens 1 Gew.-%, vorzugsweise wenigstens 5 Gew.-%, mindestens eines Polysiloxans, das vorzugsweise ausgewählt ist aus den Gruppen der linearen, verzweigten, zyklischen und vernetzten Polysiloxane, enthalten, zur Schmierung von Transportbandanlagen in der Lebensmittelindustrie, wobei die Formulierungen direkt, ohne im Lebensmittelbetrieb mit Wasser verdünnt zu werden, über eine Applikationsvorrichtung, die vorzugsweise während der Applikation in direktem Kontakt mit den zu schmierenden Oberflächen steht, oder in einer anderen bevorzugten Ausführung als Sprühvorrichtung ausgelegt ist, auf die Transportbandanlagen gelangen. In einer bevorzugten erfindungsgemäßen Verwendung enthalten die Formulierungen zusätzlich mindestens eine Komponente ausgewählt aus Fluor- und Polyhydroxyverbindungen und/oder deren Ether und Ester.

Wenn Fluorverbindungen in den erfindungsgemäß zu verwendenden Formulierungen eingesetzt werden, werden diese vorzugsweise ausgewählt aus den Gruppen der
a) per- oder teitfluorierten monomeren organischen Verbindungen,
b) reinen und gemischten Di- und Oligomeren, die auf zumindest einem per- oder teilfluorierten organischen Monomer basieren,
c) reinen und gemischten Polymeren, die auf zumindest einem per- oder teilfluorierten organischen Monomer basieren.

Bei der Definition der Grenze zwischen Oligomeren und Polymeren bezieht sich die vorliegende Erfindung auf die allgemein bekannte Charakterisierung von Polymeren, die aus so vielen gleichen oder ähnlichen niedermolekularen Bausteinen (Monomeren) aufgebaut sind, daß sich die physikalischen Eigenschaften dieser Substanzen, insbesondere die Viskoelastizität, bei Erhöhung oder Reduzierung der Anzahl der Bausteine um eine Einheit nicht mehr merklich ändem. Dies ist im allgemeinen dann der Fall, wenn die mittlere Molmasse der Polymeren' 10.000 g/mol oder mehr beträgt.

Für die niedermolekularen Dimere, Trimere und andere niedere Glieder der polymerhomologen Reihe verwendet man die Bezeichnung Oligomere.

In einer bevorzugten Form umfassen die Fluorverbindungen a) zumindest per- und teilfluorierte Tenside, Alkane, Ether und Amine, wobei besonders bevorzugt in den erfindungsgemäß verwendeten Formulierungen Ammoniumperfluoralkylsulfonate, Lithiumperfluoralkylsutfonate, Kaliumperfluoralkylsulfonate, Aminperfluoralkylsulfonate, Natriumperfluoralkylsulfonate, Kaliumfluoralkytcarboxylate, quaternäre fluorierte Alkylamomoniumiodide, Ammoniumperfluoralkylcarboxalate, fluorierte Alkylpolyoxyethylenethanole, fluorierte Alkylalkoxylate, fluorierte Alyklester mit Konzentrationen von 0,001 bis 10% zum Einsatz kommen. Die fluorierten Komponenten der Gruppe c) sind vorzugsweise per- und/oder teilfluorierte Alkoxypolymere, die besondes bevorzugt aus der Copolymerisation von Tetrafluorethylen und Perfluoralkoxyvinylethem erhältlich sind.

In einer weiterhin bevorzugten Ausführung sind in den erfindungsgemäß zu verwendenden Formulierungen aus der Gruppe c) zumindest per- und/oder teilfluorierte Polyether enthalten.

Wenn in den erfindungsgemäß zu verwendenden Formulierungen Polyhydroxyverbindungen zum Einsatz kommen, werden diese vorzugsweise ausgewählt aus den Gruppen der Polyalkohole und Kohlenhydrate, und ganz besonders bevorzugt ausgewählt aus mehrwertigen Alkoholen, vorzugsweise Alkandiole, Alkantriole, besonders bevorzugt Glycerin und den davon abgeleiteten Polyethem, sowie Glukose, Arabinose, Ribulose, Fructose und den davon abgeleiteten Oligo- und/oder Polysacchariden und deren Ester und Ether enthalten.

Es ist außerdem bevorzugt, daß die erfindungsgemäß zu verwendenden Formulierungen in Form von Ein-Komponenten-Flüssigkeiten, Lösungen, Gelen, Emulsionen, Pasten, Dispersionen vorliegen.

Die erfindungsgemäß zu verwendenden Formulierungen enthalten in einer bevor zugten Ausführung zusätzlich mindestens eine antimikrobielle Komponente ausgewählt aus den Gruppen der Alkohole, Aldehyde, antimikrobiellen Säuren, Carbonsäureester, Säureamide, Phenole, Phenolderivate, Diphenyle, Diphenylalkane, Harnstoffderivate, Sauerstoff-, Stickstoff-Acetale sowie -Formate, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, lod, lodophore, Peroxide, wobei in einer besonders bevorzugten Ausführung die erfindungsgemäß zu verwendenden Formulierungen als antimikrobielle Komponenten eine oder mehrere Verbindungen ausgewählt aus Ethanol, n-Propanol, i-Propanol, 1,3-Butandiol, Phenoxyethanol, 1,2-Propylenglykol, Glycerin, Undecylensäure, Zitronensäure, 2-Benzyl-4-chlorphenol, 2,2'-Methylen-bis-(6-brom-4-chlorphenol), 2,4,4-Trichlor-2-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octa-namin)-dihydrochlorid, N,N'-Bis-(4-Chlorphenyl)-3,12-diimino-2.4,11,13-tetraaza-tetradecandiimidamid, quaternären Ammoniumverbindungen oder Alkyl-Aminen, Guanidinen, Amphoteren, enthalten.

In einer ebenfalls bevorzugten Ausführung enthalten die erfindungsgemäß zu verwendenden Formulierungen weitere Komponenten ausgewählt aus den Gruppen der Tenside und Lösevermittter, wobei besonders bevorzugt ist, daß als Tensid zumindest ein Alkylpolyglykosid enthalten ist. Weitere bevorzugte Bestandteile sind Fettalkylamine und/oder deren Alkoxylate, insbesondere Kokosfettaminethoxylate und/oder Imidazolinverbindungen und/oder amphotere Tenside und/oder nichtionische Tenside und/oder Ethercarbonsäuren und/oder Etheraminverbindungen. Darüber hinaus ist es bevorzugt, den erfindungsgemäß zu verwendenden Formulierungen Paraffinverbindungen zuzusetzen.

Die erfindungsgemäß zu verwendenden Formulierungen werden in der Praxis auf die Transportketten aufgebracht, wobei im günstigsten Fall zu beobachten ist, daß beim Transport der Transportgüter auf den Transportaniagen kein Schaum entsteht. Es ist weiterhin bevorzugt, daß die efindungsgemäß zu verwendenden Formulierungen im Vergleich zu üblichen Schmiermitteln, die in Transportanlagen mit Wasser um einen Verdünnungsfaktor von mehr als 100 verdünnt werden und für den Fall, daß innerhalb eines definierten Zeitraums gewichtsgleiche Mengen an schmieraktiven Komponenten auf die Transportbandanlage aufgetragen werden, bei Einsatz von erfindungsgemäß zu verwendenden Formulierungen der Reibwiderstand zwischen Transportgütern und Transportbandanlage um mehr als 20 % gesenkt wird. Dies kann durch Beispielversuche gezeigt werden.

### Beispiel 1:

Eine Vergleichsformulierung 1, die 5 Gew.-% Kokospropylendiamin enthält und mit Essigsäure auf pH 7 eingestellt wird, wird über einen Düsenstock mit 5 Düsen, die je eine Sprühleistung von 5 1 pro Stunde haben, in 0,2%iger wäßriger Konzentration auf die Transportketten aufgetragen. Über einen Zeitraum von 1 Stunde gelangen somit 50 ml der Vergleichsformulierung, bzw. ca. 2,5 g des Kokospropylendiamins auf die Transportketten. Dieser Versuch wird über einen Zeitraum von 10 Stunden durchgeführt. Der Reibkoeffizient zwischen Getränkeflasche und Edelstahltransportketten wird gemäß der vorliegenden Erfindung als Verhältnis des Zuggewichts, das beispielsweise auf eine Federwaage ausgeübt wird, wenn man versucht, bei laufenden Transportketten eine Getränkeflasche festzuhalten, zu dem Gewicht der Flasche definiert.

Der Reibkoeffizient bei Verwendung des oben beschriebenen Vergleichsbeispiels µ = 0,10. Sobald man den Dosiervorgang unterbricht, steigt der Reibkoeffizient schnell an und es kommt innerhalb weniger Minuten zum Umfallen der Transportgüter bzw. der Flaschen.

Innerhalb des gesamten Versuchszeitraums von 10 Stunden gelangen bei dem Vergleichsbeispiel insgesamt 25 ml an schmieraktiven Kokospropylendiaminrohstoffen auf die Transportketten. In einem zweiten Versuch werden 25 ml einer erfindungsgemäß zu verwendenden Formulierung, bestehend aus einer 35%igen wäßrigen Polydimethylsiloxan-Dispersion, mit einem Lappen auf den Transportketten verteilt. Danach wird unter identischen Bedingungen, wie im Versuch mit Vergleichsbeispiel 1, der Reibkoeffizient zwischen Flaschen und Transportkette über einen Zeitraum von 10 Stunden gemessen. Über den gesamten Zeitraum von 10 Stunden liegt der Reibkoeffizient µ bei etwa 0,05. Dieses Beispiel zeigt, daß der Reibwiderstand zwischen Transportgütem und Transportbandanlage um mehr als 20 % gesenkt werden kann, im vorliegenden Beispiel sogar um mehr als 40 %.

Eine weitere bevorzugte Ausführung der vorliegenden Erfindung ist der Einsatz der erfindungsgemäß zu verwendenden Formulierungen für den Transport von Kunststoffgebinden, wobei die Kunststoffgebinde besonders bevorzugt mindestens ein Polymer ausgewählt aus den Gruppen der Polyethylenterephthalate (PET), Polyethylennaphthenate (PEN), Polycarbonate (PC), PVC enthalten und ganz besonders bevorzugt PET-Getränke-Flaschen sind.

### Beispiel 2:

Im Laborversuch wird die Spannungsrißkorrosion von einem Vergleichsbeispiel auf Basis von 5 Gew.-% Kokospropylendiamin und 5 Gew.% Dimethyllaurylamin, die mit Essigsäure auf pH 7 eingestellt werden, im Vergleich zur Spannungsrißkorrosion von einer 25%igen wäßrigen Polydimethylsiloxan-Dispersion gemessen.

Laut Versuchsbeschreibung werden PET-Flaschen mit Wasser gefüllt und mit Kohlendioxid so konditioniert, daß im Innenbereich der Flaschen ein Druck von etwa 7 bar vorliegt. Danach werden die Bodentassen der Flaschen in die Formulierung des Vergleichsbeispiels bzw. des erfindungsgemäß zu vewendenden Beispiels getaucht und über einen Zeitraum von 24 Stunden in eine Petri-Schale gestellt. Nach den 24 Stunden werden die Flaschen geöffnet, entleert und die Bodentassen mit Wasser abgespült. Bei visueller Auswertung der Bodentassen kann man feststellen, daß bei dem Versuch mit dem Vergleichsbeispiel viele Spannungsrisse mit mittlerer Tiefe, Einstufung C, vorliegen, während für den Versuch mit dem erfindungsgemäß zu verwendenden Beispiel festzustellen ist, daß nur wenige Spannungsrisse mit geringer Tiefe, Einstufung A, vorliegen. Die Einstufung erfolgt in Anlehnung an die Referenzbilder, die in Kapitel IV-22 des Buches 'CODE OF PRACTICE - Guidelines for an Industrial Code of Practice for Refillable PET Bottles', Edition 1, 1993-1994, enthalten sind.

Beispiel 2 zeigt daß die erfindungsgemäß zu verwendenden Formulierungen gegenüber üblichen im Markt als Schmiermittel eingesetzten Produkten auf Amin-Basis Vorteile beim Transport von Kunststoffflaschen haben.

In einer weiteren bevorzugten Ausführung werden die erfindungsgemäß zu verwendenden Formulierungen für den Transport von Kartonverpackungen verwendet. De Transportflächen der Transportbandanlage mind aus Metall, besonders bevorzugt aus Edelstahl.

Es ist weiterhin bevorzugt, den erfindungsgemäß zu verwendenden Formulierungen vor oder nach der Applikation durch separate Zuführung zusätzliche antimikrobielle Stoffe, besonders bevorzugt organische Persäuren, Chlordioxid oder Ozon, zuzusetzen.

In einer bevorzugten Ausführung werden die erfindungsgemäß zu verwendenden Formulierungen über ein Hitfsmittel, das ausgewählt sein kann aus Pinsel, Schwamm, Rollen, Tücher, Lappen, Bürsten, Wischer, Gummi, Sprühvorrichtung auf die Transportbänder ohne vorherige Verdünnung mit Wasser aufgebracht. In einer anderen bevorzugten Ausführung werden die erfindungsgemäß zu verwendenden Formulierungen in automatischen Transportbandanlagen mit Wasser verdünnt und die Anwendungslösung über Dosiervonichtungen auf die Transportbänder aufgebracht, wobei der Verdünnungsfaktor zwischen 10.000 und 100 liegt Es ist weiterhin bevorzugt, daß die erfindungsgemäß zu verwendenden Formulierungen so ausgewählt und angewandt werden, daß auf Oberflächen, die mit den Formulierungen oder Lösung in Kontakt stehen, keine weitere Vermehrung von Mikroorganismen erfolgt und ganz besonders bevorzugt, die Anzahl der Mikroorganismen reduziert wird.

Der Vorteil der erfindungsgemäßen Verwendung besteht darin, daß der Wasserverbrauch wesentlich gesenkt wird. Da die Bandschmieriösung nach dem Stand der Technik nicht aufgefangen und wiederverwendet wird, ist das heute praktizierte Verfahren mit enormer Resourcen-Verschwendung verbunden.

Ein weiterer Vorteil ist, daß kaum Mittel bei sachgemäßer Applikation auf den Boden tropfen. Dies wirkt sich aus in erhöhter Sicherheit sowie rein optischen Vorteilen im Betrieb. Außerdem wird in einigen Fällen beobachtet, daß aufkommender Schmutz von den mit dem Mittel konditionierten Transportbändern abgewiesen wird.

## Patentansprüche

1. Verwendung von Formulierungen, die bezogen auf die gesamte Formulierung wenigstens 1 Gew.-% mindestens eines Polysiloxans enthalten, zur Schmierung von Transportbandanlagen in Lebensmittelbetrieben, wobei die Formulierungen direkt, ohne im Lebensmittelbetrieb mit Wasser verdünnt zu werden, über eine Applikationsvorrichtung auf die Transportbandanlagen appliziert werden, wobei die Transportflächen der Transportbandanlage aus Metall sind.

2. Verwendung nach Anspruch 1, **dadurch gekennzeichnet, dass** die Applikationsvorrichtung während der Applikation in direktem Kontakt mit den zu schmierenden Oberflächen steht.

3. Verwendung nach Anspruch 1, **dadurch gekennzeichnet, dass** als Applikationsvonichtung eine Sprühvorrichtung verwendet wird.

4. Verwendung nach einem der Ansprüche 1 bis 3, **dadurch gekennzeichnet, dass** die Formulierungen zusätzlich mindestens eine Komponente ausgewählt aus den Fluor- und Polyhydroxyverbindungen und/oder deren Ether und Ester enthalten.

5. Verwendung nach Anspruch 4, **dadurch gekennzeichnet, dass** eine Fluorverbindung ausgewählt aus den Gruppen der
a) per- oder teilfluorierten monomeren organischen Verbindungen,
b) reinen und gemischten Dl- und Oligomeren, die auf zumindest einem per- oder teilfluorierten organischen Monomer basieren,
c) reinen und gemischten Polymeren, die auf zumindest einem per-oder teilfluorierten organischen Monomer basieren enthalten ist.

6. Verwendung nach einem der Ansprüche 4 oder 5, **dadurch gekennzeichnet, dass** als Polyhydroxyverbindungen mindestens eine Komponente ausgewählt aus mehrwertigen Alkoholen, vorzugsweise Alkandiole, Alkantriole, und den davon abgeleiteten Polyethem, sowie aus den Kohlenhydraten, vorzugsweise Glucose, Arabinose, Ribulose, Fructose und den davon abgeleiteten Oligo- und/oder Polysacchariden und deren Ester und Ether enthalten ist.

7. Verwendung nach Anspruch 6, **dadurch gekennzeichnet, dass** als Polyhydroxyverbindung zumindest Glycerin enthalten ist.

8. Verwendung nach einem der Ansprüche 1 bis 7, **dadurch gekennzeichnet, dass** die Formulierungen als Ein-Komponenten-Flüssigkeit, Lösung, Gel, Emulsion, Paste, Dispersion vorliegen.

9. Verwendung nach einem der Ansprüche 1 bis 8, **dadurch gekennzeichnet, dass** die Formulierungen zusätzlich mindestens eine antimikrobielle Komponente ausgewählt aus den Gruppen der Alkohole, Aldehyde, antimikrobiellen Säuren, Carbonsäureester, Säureamide, Phenole, Phenolderivate, Diphenyle, Diphenylalkane, Harnstoffderivate, Sauerstoff-, Stickstoff Acetale sowie -Formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, lod, lodophore, Peroxide enthalten.

10. Verwendung nach Anspruch 9, **dadurch gekennzeichnet, dass** die Formulierungen als antimikrobielle Komponenten eine oder mehrere Verbindungen ausgewählt aus Ethanol, n-Propanol, i-Propanol, 1.3-Butanidiol, Phenoxyethanol, 1,2-Propylenglykol, Glycerin, Undecylensäure, Zitronensäure, 2-Benzyl-4-chlorphenol, 2,2'-Methylen-bis-(8-brom-4-chlorphenol), 2.4,4'-Trichlor-2'-hydroxydiphenylether, N-(4-Chlorphenyl)-N-(3,4-dichlorphenyl)-hamstoff, N,N-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraaza-tetradecandiimidamid, quaternären Ammoniumverbundungen oder Alkyl-Aminien, Guanidinen, Amphoteren.

11. Verwendung nach einem der Ansprüche 1 bis 10, **dadurch gekennzeichnet, dass** die Formulierungen zusätzliche Komponenten ausgewählt aus den Gruppen der Tenside und Lösevermittler enthalten.

12. Verwendung nach einem der Ansprüche 1 bis 11 für den Transport von Kunststoff-Gebinden.

13. Verwendung nach Anspruch 12, **dadurch gekennzeichnet, dass** die Kunststoff-Gebinde mindestens ein Polymer ausgewählt aus den Gruppen der Polyethylenterephthalate (PET), Polyethylennaphtenate (PEN), Polycarbonate (PC), PVC enthalten.

14. Verwendung nach Anspruch 13, **dadurch gekennzeichnet, dass** die Kunststoff-Gebinde PET-Getränke-Flaschen sind.

15. Verwendung nach einem der Ansprüche 1 bis 11 für den Transport von Kartonverpackungen.

16. Verwendung nach einem der Ansprüche 1 bis 15, **dadurch gekennzeichnet, dass** die Transportflächen der Transportbandanlage aus Kunststoff sind.

17. Verwendung nach einem der Ansprüche 1 bis 16, **dadurch gekennzeichnet, dass** bei der Applikation separat zusätzliche antimikrobielle Wirkstoffe zugesetzt werden.

18. Verwendung nach Anspruch 17, **dadurch gekennzeichnet, dass** als antimikrobielle Wirkstoffe organische Persäuren, Chlordioxid oder Ozon verwendet werden.

## Claims

1. Use of formulations, which, based on the total formulation, include at least 1 wt.% of at least one polysiloxane for lubricating belt conveyor systems in food manufacturing plants, wherein the formulations, without being diluted with water in the food manufacturing plant, are applied directly to the belt conveyor systems using an applicator device, the conveyor surfaces of said belt conveyor system being made of metal.

2. The use as claimed in claim 1, **characterized in that** the applicator device during application is in direct contact with the surfaces to be lubricated.

3. The use as claimed in claim 1, **characterized in that** a spray device is used as applicator device.

4. The use as claimed in any of claims 1 to 3, **characterized in that** the formulations additionally include at least one component selected from among fluorine compounds and polyhydroxy compounds and/or ethers and esters thereof.

5. The use as claimed in claim 4, **characterized in that** a fluorine compound selected from the groups of
a) perfluorinated or partially fluorinated monomeric organic compounds,
b) pure and mixed di- and oligomers based on at least one perfluorinated or partially fluorinated organic monomer,
c) pure and mixed polymers based on at least one perfluorinated or partially fluorinated organic monomer
is included.

6. The use as claimed in any of claims 4 or 5, **characterized in that** at least one component selected from polyhydric alcohols, preferably alkanediols, alkanetriols and polyethers derived therefrom, as well as carbohydrates, preferably glucose, arabinose, ribulose, fructose and oligo- and/or polysaccharides derived therefrom and esters and ethers thereof is included as polyhydroxy compound.

7. The use as claimed in claim 6, **characterized in that** at least glycerol is included as polyhydroxy compound.

8. The use as claimed in any of claims 1 to 7, **characterized in that** the formulations are in the form of a single-component liquid, solution, gel, emulsion, paste, dispersion.

9. The use as claimed in any of claims 1 to 8, **characterized in that** the formulations additionally include at least one antimicrobial component selected from the groups of alcohols, aldehydes, antimicrobial acids, carboxylic esters, acid amides, phenols, phenol derivatives, diphenyls, diphenylalkanes, urea derivatives, oxygen acetals, nitrogen acetals as well as formals, benzamidines, isothiazolines, phthalimide derivatives, pyridine derivatives, antimicrobial surface-active compounds, guanidines, antimicrobial amphoteric compounds, quinolines, 1,2-dibromo-2,4-dicyanobutane, iodo-2-propynylbutyl carbamate, iodine, iodophores, peroxides.

10. The use as claimed in claim 9, **characterized in that** the formulations include as antimicrobial components one or more compounds selected from ethanol, n-propanol, isopropanol, 1,3-butanediol, phenoxyethanol, 1,2-propylene glycol, glycerol, undecylenic acid, citric acid, 2-benzyl-4-chlorophenol, 2,2'-methylene-bis(6-bromo-4-chlorophenol), 2,4,4'-trichloro-2'-hydroxydiphenyl ether, N-(4-chlorophenyl)-N-(3,4-dichlorophenyl)urea, N,N'-(1,10-decanediyldi-1-pyridinyl-4-ylidene)-bis(1-octaneamine) dihydrochloride, N,N'-bis-(4-chlorophenyl)-3,12-diimino-2,4,11,13-tetraazatetradecane diimide amide, quaternary ammonium compounds or alkylamines, guanidines, amphoteric substances.

11. The use as claimed in any of claims 1 to 10, **characterized in that** the formulations include additional components selected from the groups of surfactants and solubilizers.

12. The use as claimed in any of claims 1 to 11 for conveying plastic containers.

13. The use as claimed in claim 12, **characterized in that** the plastic containers include at least one polymer selected from the groups of polyethylene terephthalates (PET), polyethylene naphthenates (PEN), polycarbonates (PC), PVC.

14. The use as claimed in claim 13, **characterized in that** the plastic containers are PET beverage bottles.

15. The use as claimed in any of claims 1 to 11 for conveying carton packages.

16. The use as claimed in any of claims 1 to 15, **characterized in that** the conveyor surfaces of said belt conveyor system are made of plastic material.

17. The use as claimed in any of claims 1 to 16, **characterized in that** application involves separate addition of further antimicrobial agents.

18. The use as claimed in claim 17, **characterized in that** organic peracids, chlorine dioxide or ozone are used as antimicrobial agents.

## Revendications

1. Utilisation de formulations, qui contiennent, par rapport à la formulation totale, au moins 1 % en poids d'au moins un polysiloxane en vue de la lubrification d'installations de bandes transporteuses dans des entreprises de produits alimentaires, sachant que les formulations sont appliquées sur les installations de bandes transporteuses, directement, sans avoir à être diluées à l'aide d'eau dans l'entreprise de produits alimentaires, les surfaces de transport des installations de bandes transporteuses étant en métal.

2. Utilisation selon la revendication 1, **caractérisée en ce que** le dispositif d'application se trouve en contact direct avec les surfaces à lubrifier.

3. Utilisation selon la revendication 1, **caractérisée en ce que** l'on utilise, en tant que dispositif d'application, un dispositif à pulvérisation.

4. Utilisation selon l'une quelconque des revendications 1 à 3, **caractérisée en ce que** les formulations contiennent en sus au moins un composant sélectionné parmi les composés fluorés et polyhydroxylés et/ou leurs éthers et esters.

5. Utilisation selon la revendication 4, **caractérisée en ce qu'**est contenu un composé fluoré sélectionné parmi les groupes
a) des composés organiques monomères perfluorés ou partiellement fluorés,
b) des dimères et oligomères purs et mixtes, qui se basent sur tout au moins un monomère organique perfluoré ou partiellement fluoré,
c) des polymères purs et mixtes, qui se basent sur tout au moins un monomère organique perfluoré ou partiellement fluoré.

6. Utilisation selon l'une quelconque des revendications 4 ou 5, **caractérisée en ce qu'**est contenu, en tant que composés polyhydroxylés, au moins un composant sélectionné parmi les alcools polyhydriques, de préférence les alcanediols, les alcanetriols et les polyéthers qui en dérivent, ainsi que les glucides, de préférence le glucose, l'arabinose, le ribulose, le fructose et les oligosaccharides et/ou polysaccharides qui en dérivent et leurs esters et éthers.

7. Utilisation selon la revendication 6, **caractérisée en ce qu'**est contenu, en tant que composé polyhydroxylé, au moins la glycérine.

8. Utilisation selon l'une quelconque des revendications 1 à 7, **caractérisée en ce que** les formulations sont présentes en tant que liquide, solution, gel, émulsion, pâte, dispersion à un composant.

9. Utilisation selon l'une quelconque des revendications 1 à 8, **caractérisée en ce que** les formulations contiennent en sus au moins un composant antimicrobien sélectionné parmi les groupes des alcools, des aldéhydes, des acides antimicrobiens, des carboxylates, des amides d'acide, des phénols, des dérivés du phénol, des diphényles, des diphénylalcanes, des dérivés uréiques, des acétals et formaldéhydes à oxygène et azote, des benzamidines, des isothiazolines, des dérivés de phtalimide, des dérivés de pyridine, des composés tensioactifs antimicrobiens, des guanidines, des composés amphotères antimicrobiens, des quinoléines, du 1,2-dibromo-2,4-dicyanobutane, de l'iodo-2-propynylbutyl-carbamate, de l'iode, des iodophores, des peroxydes.

10. Utilisation selon la revendication 9, **caractérisée en ce que** les formulations contiennent, en tant que composant antimicrobien, un ou plusieurs composés sélectionnés parmi l'éthanol, le n-propanol, l'iso-propanol, le 1,3-butanediol, le phénoxyéthanol, le 1,2-propylèneglycol, la glycérine, l'acide undécylénique, l'acide citrique, le 2-benzyl-4-chlorophénol, le 2,2'-méthylène-bis-(6-bromo-4-chlorophénol), l'éther 2,4,4'-trichloro-2'-hydroxydiphénylique, la N-(4-chlorophényl)-N-(3,4-dichlorophényl)-urée, le dihydrochlorure de N,N'-(1,10-décanediyl-di-1-pyridinyl-4-ylidène)-bis-(1-octanamine), le N,N'-bis-(4-chlorophényl)-3,12-diimino-2,4,11,13-tétraaza-tétradécane-diimide-amide, des composés ammonium quaternaires ou des alkylamines, des guanidines, des amphotères.

11. Utilisation selon l'une quelconque des revendications 1 à 10, **caractérisée en ce que** les formulations contiennent des composés supplémentaires sélectionnés parmi les groupes des agents tensioactifs et des agents solubilisants.

12. Utilisation selon l'une quelconque des revendications 1 à 11 pour le transport de fûts en matière synthétique.

13. Utilisation selon la revendication 12, **caractérisée en ce que** les fûts en matière synthétique contiennent au moins un polymère sélectionné parmi les groupes des téréphtalates de polyéthylène (PET), des naphténates de polyéthylène (PEN), des polycarbonates (PC), du PVC.

14. Utilisation selon la revendication 13, **caractérisée en ce que** les fûts en matière synthétique sont des bouteilles pour boissons en PET.

15. Utilisation selon l'une quelconque des revendications 1 à 11 pour le transport d'emballages en carton.

16. Utilisation selon l'une quelconque des revendications 1 à 15, **caractérisée en ce que** les surfaces de transport des installations de bandes transporteuses sont en matière synthétique.

17. Utilisation selon l'une quelconque des revendications 1 à 16, **caractérisée en ce que**, lors de l'application, l'on ajoute séparément des agents antimicrobiens supplémentaires.

18. Utilisation selon la revendication 17, **caractérisée en ce que** l'on utilise, en tant qu'agents actifs antimicrobiens, des peracides organiques, le dioxyde de chlore ou l'ozone.
